(19) Europäisches Patentamt
European Patent Office
Office européen des brevets

(11) Publication number: 0 154 103
B1

(12) # EUROPEAN PATENT SPECIFICATION

(45) Date of publication of patent specification: **27.01.88**

(51) Int. Cl.$^4$: **A 61 F 2/04,** A 61 L 27/00

(21) Application number: **84830317.8**

(22) Date of filing: **23.11.84**

(54) **Physiologically absorbable prosthesis for anastomosis of vessels and canals of the human and animal organism.**

(30) Priority: **22.02.84 IT 4773484**

(43) Date of publication of application:
**11.09.85 Bulletin 85/37**

(45) Publication of the grant of the patent:
**27.01.88 Bulletin 88/04**

(84) Designated Contracting States:
**AT BE CH DE FR GB IT LI LU NL SE**

(56) References cited:
**DE-A-2 034 413**

(73) Proprietor: **Di Mezza, Antonio**
**Via Piana 24**
**I-82037 Telese (Benevento) (IT)**

(72) Inventor: **Di Mezza, Antonio**
**Via Piana 24**
**I-82037 Telese (Benevento) (IT)**

(74) Representative: **Bazzichelli, Alfredo et al**
**c/o Società Italiana Brevetti Piazza Poli 42**
**I-00187 Roma (IT)**

EP 0 154 103 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a surgical prosthesis for anastomosis of blood vessels, and canals in general, in humans and animals, which is absorbed physiologically by the organism after healing of the connected vessels or canals.

Although the present invention has been developed for and is particularly directed towards the field of vascular microsurgery, it may be applied in a more general way to extend to the anastomosis of any canal structure in the organism.

Microsurgery has recently developed more and more rapidly, due in part to the progress achieved by the technical aids, such as dioptical enlargement devices, instruments, and bipolar coagulation. Although progress has also been made with regard to the suture materials, sutures are still performed using traditional methods of application of the stitches, even in the case of blood vessels of very small diameter, in the order of 1 mm or less. The traditional methods of suture are very difficult in the field of microsurgery: they are laborious, time-consuming and moreover do not provide the necessary certainty of achieving the desired aim, that is, the perfect healing of the connection of the blood vessels which were resected, as well as the correct flow of fluid through the vessel.

German publication DE—A—2034413 describes a small tube for anastomosis, having two tapered ends and grooves on its outer surface. Blood vessels to be joined are inserted onto both ends of the tube and jointed at the middle of the tube. Clamping rings secure the vessels in the grooves of the tube. The tube is made of saccarose as a primary component, which is finally dissolved in the flowing blood.

The prosthesis of the present invention comprises a pair of end members on which the two ends of the vessels are to be fixed and a central member which retains the end members and the vessels by mechanical friction against the central member, which encircles the vessels for a substantial portion. This provides the advantage of supporting a portion of the vessels in the junction area in a rigid and continuous manner, both internally and externally. Moreover the junction of the vessels is not made at the resected sections, but rather in an untouched area of the same vessels.

An aim of the present invention is to provide a prosthesis for the anastomosis of canals in general and/or microvascular anastomosis in particular, which eliminates the need for suturing the vessels with stitches and uses a prosthesis easy to apply and of high reliability.

Another aim of the present invention, in addition to the former, is to provide a prosthesis which is absorbed by the organism within a sufficient period of time to allow healing of the connection of the canals, so as to leave no extraneous bodies in the organism.

By means of the prosthesis according to the present invention, an end-to-end prosthesis may be performed, while with a slightly modified prosthesis, an end-to-side prosthesis may be carried out.

The prosthesis for end-to-end anastomosis comprises three members: a central tubular member, and two end members, into each of which the two ends of the vessel or canal to be connected are inserted, said vessel being turned back over the outside of the end members, which are then inserted into the central member until they touch, the central member maintaining the connection and ensuring that the blood or other fluid is sealed inside the vessel.

In sum, the prosthesis comprises: an elongate central member of substantially tubular shaped and with a longitudinal passage of circular section extending therethrough, characterised in that longitudinal slits extend from each end of said central member toward the mid-line thereof so as to form at each end a plurality of circumferentially spaced fingers, the inner surface of these fingers being so shaped to frictionally engage the wall of a vessel or canal, and in that it further comprises two end members of substantially tubular shape and with a longitudinal passage of circular section extending therethrough, each end member being adapted to be inserted into the opposite ends of said central member and to receive in the longitudinal passage thereof an end of a vessel or canal to be anastomosed, said end of vessel or canal being further turned over the outer surface of said end member, said outer surface of said end member having a first terminal portion so shaped as to frictionally engage the turned end of the vessel or canal and, in use, to clamp it against the inner surface of the fingers of the central member, a second portion of truncated conical shape which can co-operate with the inner surface of said fingers, and a third portion of enlarged diameter which can provide a grip for the operator, the central member and the end members being made of a physiologically absorbable co-polymer of L-lactic acid and glycolic acid, of formula

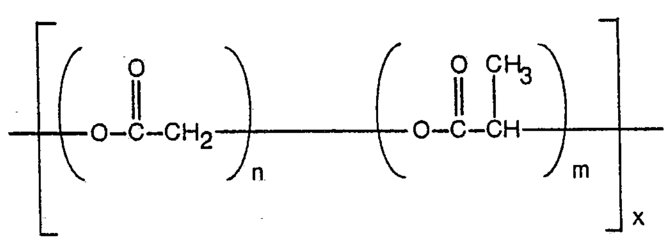

glycolic acid L—lactic acid

in which x is between 100 and 1000, and the ratio m:n is between 80:20 and 100:10.

Another object of the present invention is a modification of the prosthesis described above, in which a transverse opening is provided in the central member, to effect a side-to-end anastomosis.

The invention will be illustrated with reference to the attached drawings, in which:

Figure 1 is a side view, partially in cross-section, of the central member;

Figure 2 is a front view of the central member;

Figure 3 is a side view of an end member;

Figure 4 is a front view of an end member;

Figure 5 is a modification of the central member for a side-to-end anastomosis;

Figure 6 is a front view of the member in Figure 5;

Figures 7 and 8 show respectively the side and front views of an end member to be used in combination with the central member of Figure 5;

Figure 9 shows the steps in executing an end-to-end anastomosis;

Figure 10 shows the prosthesis members prepared for a side-to-end anastomosis.

With reference to Figures 1 and 2, the central member of the prosthesis indicated with 1 has a tubular shape with a longitudinal passage 2. At each end of central member 1 slits are provided which form a plurality of circumferentially spaced fingers 4, which extend to a certain distance from said end. The inner surface of the fingers 4 is shaped, as shown in Figure 1, with circumferential ribbing 5.

With reference to Figures 3 and 4, the end members of the prosthesis indicated with 6 have a longitudinal passage 7. The outer surface of the end member 6 has a first terminal portion 8, having, for example, a saw-toothed section, a second portion 9 of truncated conical shape and a third portion 10 of enlarged diameter.

With reference to Figures 1 to 4 and to Figure 9, where (a) indicates the relative position which the central member 1 and end members 6 must assume, the vessel or canal to be connected, indicated with 11, is inserted into the passage 7 of the end member 6 starting from the third portion 10 until it extends a certain distance in front of the first terminal portion 8. The exten ding section is turned over and placed over the portion 8, as shown in Figure 9 (b).

The end member 6 is then inserted into passage 2 of the central member 1 until the conical portion 9 abuts on the inner surface of the fingers 4. The first terminal portions 8 of the end members 6 are engaged with the surface of the longitudinal passage 2 of the central member, thus fixing in position by frictional engagement the two ends of the vessel to be anastomised inside the central member 1. Said ends are then placed in contact with one another, in correspondence with the mid-line of the central member 1, to allow their healing and connection, as shown in Figure 9 (c).

In this way, it is clear how inside the central member 1 the blood vessel forms a continuum, ensuring the flow of the biological fluid inside it.

The above description shows how the anastomosis operation is extremely rapid and reliable with respect to the previous technique of suturing the ends of the vessel with stitches. The advantage of the present invention is particularly striking in microsurgery, since the prosthesis allows an end-to-end anastomosis only a few millimeters in diameter to be performed in a few minutes by taking advantage of the physiological healing, which with usual techniques even in expert hands requires much longer times and does not always allow secure canalization of the treated vessels. In fact, usual techniques can give rise to complications like stenosis, thrombosis, etc., which are completely absent with application of the prosthesis according to the invention. The healing process occurs thanks to the particular geometry of the prosthesis which allows the two ends of the vessel to be held intimately together.

The central member allows the ends to be held in place thanks to the anchoring effect arising from the particular shape of the inner surfaces coupled with the considerable elasticity of the copolymer.

Thanks to their particular design, the end parts allow the immediate turning over of the vessel, which at the same time is held by the notches formed by the saw tooth section on portion 8.

The size and proportions are devised above all as a function of the absorption by the organism in a period of time ranging from 60 to 90 days after the operation.

Figures 5 to 8 and 10 show a modification for side-to-end microvascular prosthesis.

As shown in Figure 5, the central member of the prosthesis indicated with 1′, has in correspondence with its mid-line an opening 13 going into a transverse cylindrical conduit 14 with substantially the same characteristics of slits and fingers 15 described above for the central member of Figure 1. The conduit 14 is designed to receive a side member 16 with the same characteristics as terminal member 6 illustrated in Figures 3 and 4 and which has the same functions, except that its diameter is smaller.

The terminal members 6′ (Figures 7 and 8) for the side-to-end prosthesis have the same characteristics as the end members 6 previously illustrated in Figures 3 and 4, with the difference that in correspondence with the front end of the inner passage 7′ there is a bevelling 19 so that, once the end members 6′ are inserted in the central member 1′, a hollow space may be created between the ends of the vessels held by the end members 6′ and the end of the vessels held by the side member 16. In this way, healing of the vessels in an end-to-end position and in a lateral position establishes the connection.

Since the geometry of the members of the prosthesis in this case require precise orientation of the end members 6′ with respect to the central member 1′, a locating lug 20 is provided on the central member 12 in a position opposite to opening 13, as well as locating lugs 21 on the end members 17, in a position opposite to bevelling 19.

Figure 10 shows the members of the side-to-end prosthesis in position for the connection to be made. The side-to-end microvascular prosthesis thus serves to connect vessels of various diameter.

To appreciate the importance of the present invention, it must be considered that its utility is directed particularly to microsurgery. As an example for end-to-end prosthesis, the central member may have a length of approximately 5.5 mm and a diameter of 3.0 mm, while the terminal members have a length of 4 mm and a diameter of 3 mm in the widened portion which serves as the surgeon's grip, with a diameter of only 1 mm for the inner passage.

As an example, the prosthesis for side-to-end anastomosis may have a side member with the above mentioned dimensions for a 1 mm vessel, end members with a 3 mm inner passage, and consequent proportions of the central member.

However the prosthesis according to the invention is not limited to microsurgery, even if this is the field in which it is most advantageous, but may also be used in all types of vascular surgery, transplants and organ surgery.

The end-to-end prosthesis has already been applied experimentally in the following types of operation: kidney transplants, testes auto-transplant, porto-renal anastomosis, liver transplant, arterial and venal transplant, microsurgery of the sedimentary branches of the renal artery, etc.

The side-to-end prosthesis has already been applied experimentally in the side-to-end homolateral spermatic-large saphena anastomosis for the correction of variococele, and may be applied in all vascular situations requiring side-to-end anastomosis (when the vessels to be sutured are of different diameters), thus greatly reducing operating times and allowing a stable connection of the treated vessels.

With regard to the material of which the prosthesis is made, as mentioned above, it consists of a L-lactic and glycolic acid in the proportions indicated. This is a non-toxic material which is biodegraded in the organism within 60—90 days. The purity of the material must be very high, and is determined by differential calorimetric scanning. The chemical composition of the preparation is checked by IR and PMR spectral analysis.

The copolymer is prepared by known methods.

**Claims**

1. Physiologically absorbable prosthesis for anastomosis of vessels and canals (11) of humans and animals, comprising an elongate central member (1) of substantially tubular shape and with a longitudinal passage (2) of circular section extending therethrough, characterised in that longitudinal slits (3) extend from each end of said central member (1) toward the mid-liine thereof so as to form at each end a plurality of circumferentially spaced fingers (4), the inner surface (5) of these fingers (4) being so shaped to frictionally engage the well of a vessel or canal, and in that it further comprises two end members (6) of substantially tubular shape and with a longitudinal passage (7) of circular section extending therethrough, each end member (6) being adapted to be inserted into the opposite ends of said central member (1) and to receive in the longitudinal passage (7) thereof an end of a vessel or canal to be anastomosed, said end of vessel or canal being further turned over the outer surface of said end member (6), said outer surface of said end member having a first terminal portion (8) so shaped as to frictionally engage the turned end of the vessel or canal and, in use, to clamp it against the inner surface (5) of the fingers (4) of the central member (1), a second portion (9) of truncated conical shape which can co-operate with the inner surface (5) of said fingers (4), and a third portion (10) of enlarged diameter which can provide a grip for the operator, the central member (1) and the end members (6) being made of a physiologically absorbable co-polymer of L-lactic acid and glycolic acid, of formula

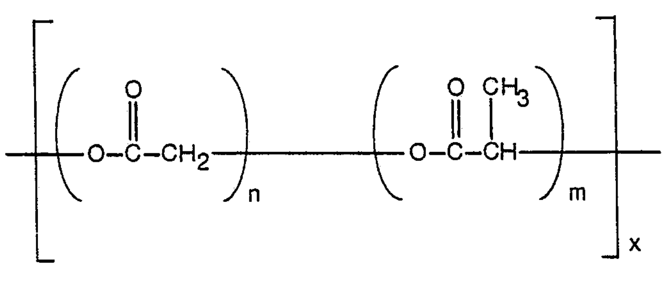

glycolic acid L—lactic acid

in which x is between 100 and 1000, and the ratio m:n is between 80:20 and 100:10.

2. Prosthesis according to claim 1, in which said portion (8) of the outer surface of the end members (6) is saw-toothed.

3. Prosthesis according to claim 1 or 2, characterised in that said central member (1') has a circular opening (13) centered on its mid-line, said opening (13) communicating with a transverse tubular conduit (14) which at one end is attached to the outer wall of the central member (1') and at the opposite end has longitudinal slits forming circumferentially spaced fingers (15), and in that it further comprises a side

member (16) having all the features of shape of the end member (6') and adapted to be inserted into the free end of said tubular conduit (14) to effect a side-to-end connection, and in that said end members (6') have a bevelling (19) at the end portion (8') of the outer surface, so as to permit communication between said transverse conduit (14) and the longitudinal passage (7') of the end members (6').

4. Prosthesis according to claim 3, in which said central member (1') and said side members (6') are equipped with locating lug (20, 21) opposite said opening (13) and said bevelling (19).

**Patentansprüche**

1. Physiologisch absorbierbare Prothese für die durchgängige Verbindung von Gefäßen und Kanälen (11) von Menschen und Tieren, enthaltend ein längliches Mittelstück (1) von im wesentlichen tubusförmiger' Gestalt und mit einem sich hindurch erstreckenden Längskanal (2) von kreisförmigem Querschnitt, dadurch gekennzeichnet, daß Längsschlitze (3) sich von jedem Ende des Mittelstücks (1) gegen die Mittenlinie desselben erstrecken, um an jedem Ende mehrere in Umfangsrichtung verteilte Finger (4) auszubilden, wobei die Innenseite (5) dieser Finger (4) so gestaltet ist, daß sie an der Wand eines Gefäßes oder Kanals mit Reibung anliegen, und daß sie weiterhin zwei Endstücke (6) von im wesentlichen tubusförmiger Gestalt und mit einem sich hindurch erstreckenden Längsdurchgang (7) von kreisförmigem Querschnitt enthält, wobei jedes Endstück (6) dazu eingerichtet ist, in die gegenüberliegenden Enden des Mittelstücks (1) eingesetzt zu werden und in seinem Längsdurchgang (7) ein Ende eines zu verbindenden Gefäßes oder Kanals aufzunehmen, wobei das Ende des Gefäßes oder Kanals weiterhin über die Außenseite des genannten Endstücks (6) umgelegt wird, die Außenseite des genannten Endstücks einen ersten Anschlußabschnitt (8) aufweist, der so gestaltet ist, daß er mit Reibung an dem umgelegten Ende des Gefäßes oder Kanals anliegt und im Gebrauch dieses gegen die Innenseite (5) der Finger (4) des Mittelstücks (1) andrückt und weiterhin einen zweiten Abschnitt (9) von kegelstumpfförmiger Gestalt, der mit der Innenseite (5) der Finger (4) zusammenwirkt, sowie einen dritten Abschnitt (10) von vergrößertem Durchmesser aufweist, der einen Griff für den Operateur bilden kann, wobei das Mittelstück (1) und die Endstücke (6) aus einem physiologisch absorbierbaren Copolymer von L-Milchsäure und Glykolsäure der Formel

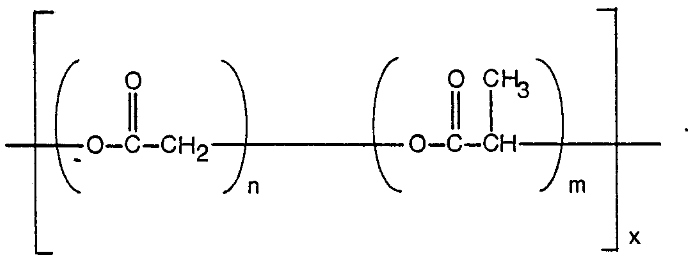

Glykolsäure L–Milchsäure

bestehen, wobei x zwischen 100 und 1000 liegt und das Verhältnis m:n zwischen 80:20 und 100:10 liegt.

2. Prothese nach Anspruch 1, bei der der genannte Abschnitt (8) der Außenseite der Endstücke (6) sägezahnförmig ist.

3. Prothese nach Anspruch 1 oder 2, dadurch gekennzeichnet, daß das Mittelstück (1') eine kreisförmige Öffnung (13) hat, die in ihrer Mittellinie zentriert ist, wobei die Öffnung (13) mit einer tubusförmigen Querleitung (14) in Verbindung steht, die an ihrem einen Ende an der Außenwand des Mittelstücks (1') befestigt ist und an ihrem entgegengestzten Ende Längsschlitze aufweist, die in Umfangsrichtung verteilte Finger (15) ausbilden, und daß sie weiterhin ein Seitenstück (16) aufweist, das sämtliche Merkmale oder der Gestalt des Endstücks (6') hat und dazu eingerichtet ist, in das freie Ende der tubusförmigen Leitung (14) eingesetzt zu werden, um eine Seite-an-End-Verbindung herzustellen, und daß die Endstücke (6') eine Abschrägung (19) am Endabschnitt (8') der Außenseite aufweisen, um eine Verbindung zwischen der Querleitung (14) und dem Längskanal (7') der Endstücke (6') zu ermöglichen.

4. Prothese nach Anspruch 3, bei der das Mittelstück (1') und die Seitenstücke (6') mit Haltenasen (20, 21) gegenüber der Öffnung (13) und der Abschrägung (19) versehen sind.

**Revendications**

1. Prothèse physiologiquement absorbable pour l'anastomose de vaisseaux et de canaux (11) chez l'homme et l'animal comprenant un élément central allongé (1) de forme substantiellement tubulaire et avec un passage longitudinal (2) le traversant de part en part de section circulaire, caractérisée en ce que des fentes longitudinales (3) s'étendent de chaque extrémité du dit élément central (1) vers sa ligne médiane, de façon à former à chaque etrémité, de nombreux doigts (4) espacés circonférentiellement, la surface intérieure (5) des doigts (4) ayant une forme telle d'engager par frottement la paroi d'un vaisseau ou canal et en ce qu'elle comprend ultérieurement deux éléments d'extrémité (6) de forme substantiellement tubulaire et avec un passage longitudinal (7) de section circulaire le traversant de part en

part, chaque élément d'extrémité (6) pouvant être introduit dans les extrémités opposées du dit élément central (1) et recevoir dans son passage longitudinal (7) une extrémité d'un vaisseau ou canal à anastomoser, la dite extrémité de vaisseau ou canal étant en outre repliée sur la surface extérieure de l'élément d'extrémité (6), la dite surface extérieure du dit élément d'extrémite ayant une première partie terminale (8) formée de façon à engager par frottement l'extrémité repliée du vaisseau ou canal et de la serrer à l'usage, contre la surface intérieure (5) des doigts (4) de l'élément central (1), une deuxième partie (9) de forme troncoconique apte a coopérer avec la surface intérieure (5) des doigts (4), et une troisième partie (10) de plus grand diamètre apte a fournir une prise à l'opérateur, l'élément central (1) et les éléments (6) d'extrémité étant formés d'un copolymère physiologiquement absorbable d'acide L-lactique et d'acide glycolique, de formule:

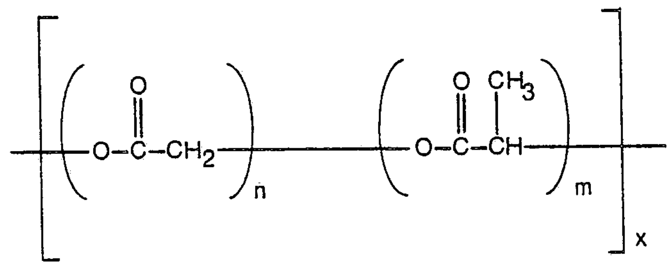

acide glycolique          acide L—lactique

où x est compris entre 100 et 1000 et le rapport m:n est compris entre 80:20 et 100:10.

2. Prothèse selon la revendication 1, où la partie (8) de la surface extérieure des éléments d'extrémité (6) est en dents de scie.

3. Prothèse selon le revendication 1 ou 2, caractérisée en ce que le dit élément central (1') a une ouverture circulaire (13) centrée sur sa ligne médiane, l'ouverture (13) communiquant avec un conduit tubulaire transversal (14) qui est attaché par une extrémité à la paroi extérieure de l'élément central (1') et dans l'extrémité opposée il a des fentes longitudinales qui forment des doigts (15) espacés circonférentiellement, et en ce que elle comprend aussi un élément latéral (16) ayant toutes les caractéristiques de forme de l'élément d'extrémité (6') et apte à être introduit dans l'extrémité libre du dit conduit tubulaire (14) pour effectuer une connexion latero-terminale, et en ce que les dits éléments d'extrémité (6') ont un biseautage (19) dans la partie d'extrémité (8') de la surface extérieure, de façon à permettre une communication entre le conduit transversal (14) et le passage longitudinal (7') des éléments d'extrémité (6').

4. Prothèse selon la revendication 3, où l'élément central (1') et les éléments latéraux (6') sont pourvus de languettes de positionnement (20, 21) à l'opposé de la dite ouverture (13) et du dit biseautage (19).

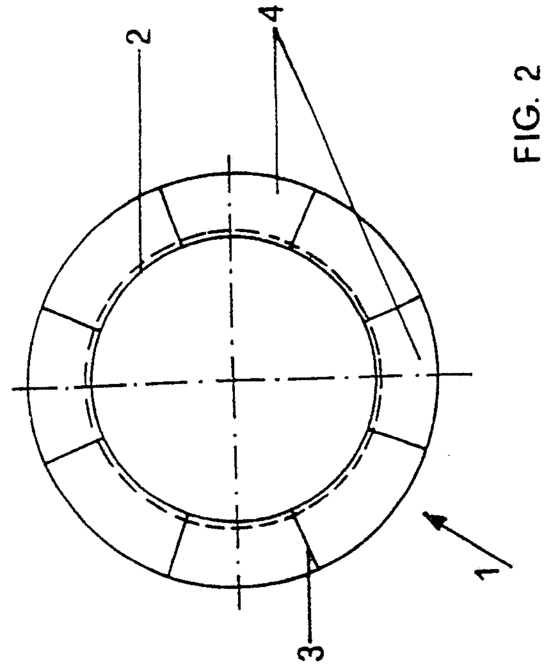


FIG. 2

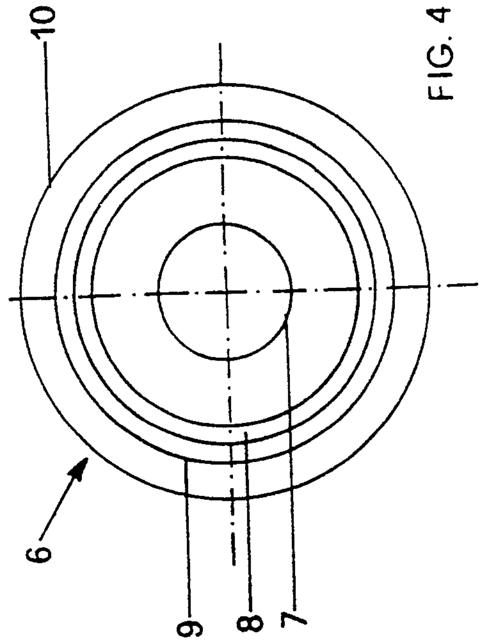


FIG. 4

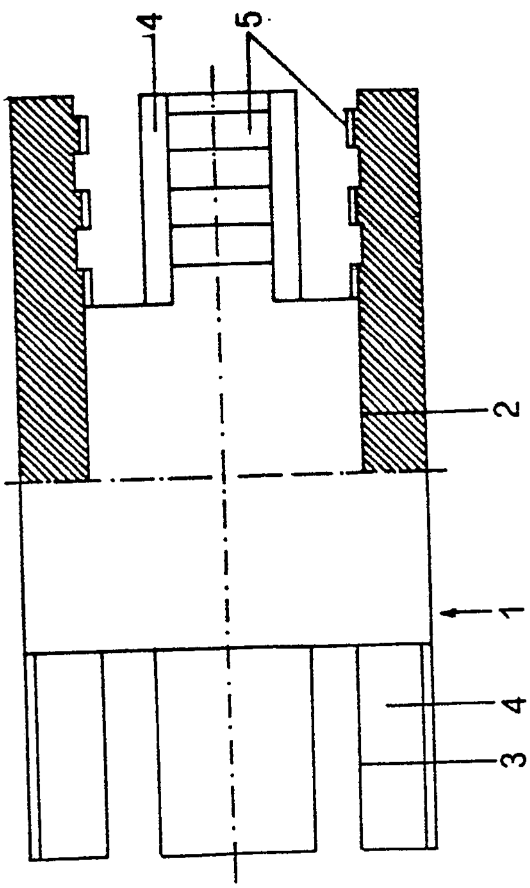


FIG. 1

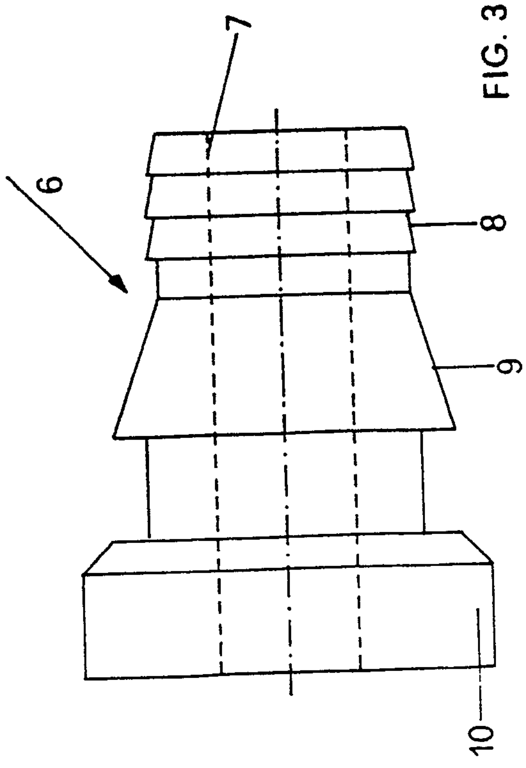


FIG. 3

FIG. 7

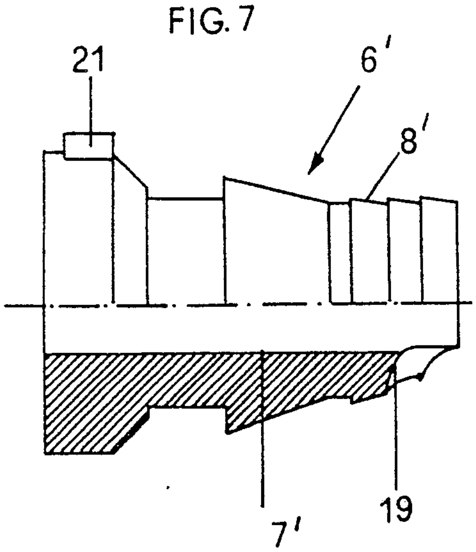


FIG. 5

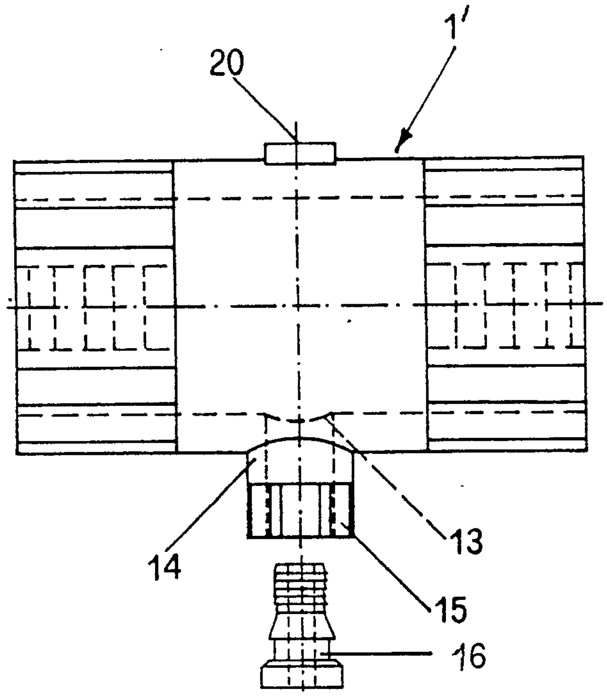


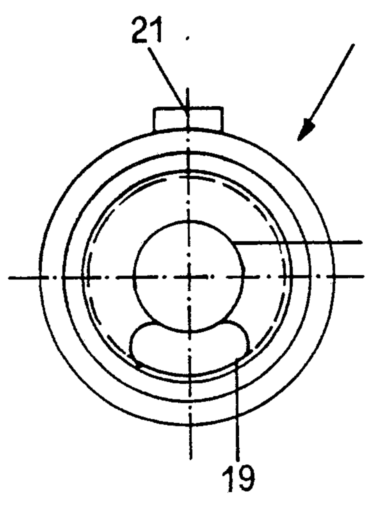


FIG. 8

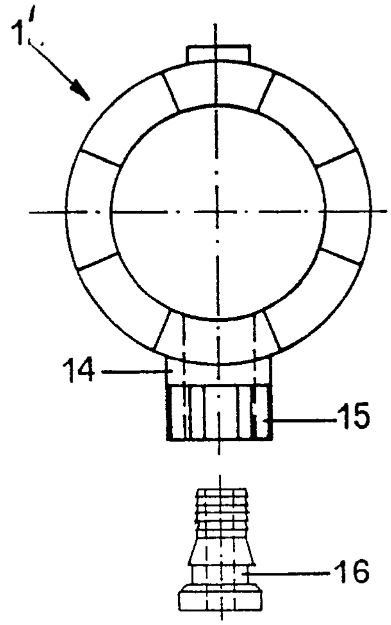


FIG. 6

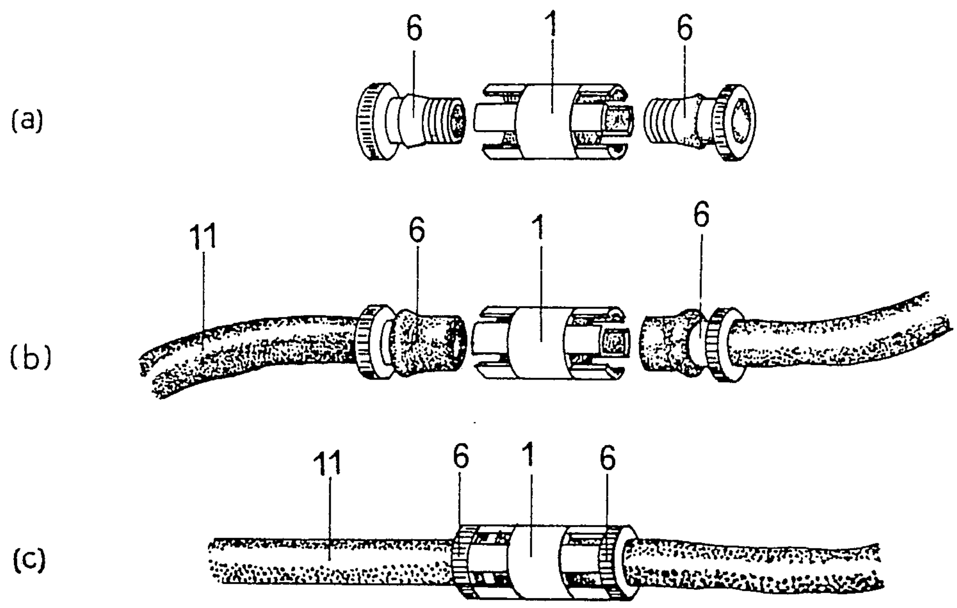


FIG. 9

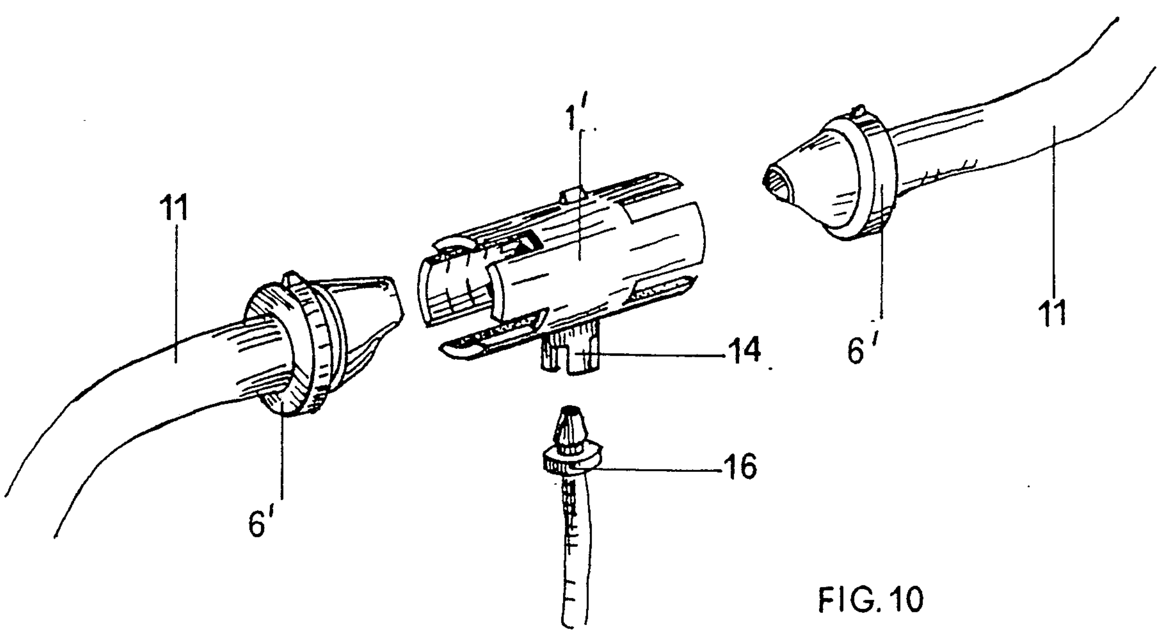


FIG. 10